(19) Europäisches Patentamt
European Patent Office
Office européen des brevets

(11) Veröffentlichungsnummer: **0 079 037**
**B1**

(12) **EUROPÄISCHE PATENTSCHRIFT**

(45) Veröffentlichungstag der Patentschrift: **29.10.86**

(51) Int. Cl.[4]: **C 07 D 251/70**, C 07 C 45/86

(21) Anmeldenummer: **82110116.9**

(22) Anmeldetag: **03.11.82**

(54) **Neue Bismelamine.**

(30) Priorität: **05.11.81 DE 3143920**

(43) Veröffentlichungstag der Anmeldung: **18.05.83 Patentblatt 83/20**

(45) Bekanntmachung des Hinweises auf die Patenterteilung: **29.10.86 Patentblatt 86/44**

(84) Benannte Vertragsstaaten: **AT BE CH DE FR GB IT LI NL SE**

(56) Entgegenhaltungen:
**DE - A - 2 625 399**
**DE - A - 2 919 496**
**US - A - 2 237 092**
**US - A - 2 544 071**

(73) Patentinhaber: **Degussa Aktiengesellschaft, Weissfrauenstrasse 9, D-6000 Frankfurt am Main 1 (DE)**

(72) Erfinder: **Werle, Peter, Dr. Dipl.-Chem., Im Börner 43, D-6460 Gelnhausen (DE)**
Erfinder: **Focke, Holger, Dr. Dipl.-Chem., Erlenweg 28, D-6454 Bruchköbel (DE)**
Erfinder: **Popp, Klaus, Talstrasse 45, D-6458 Rodenbach 2 (DE)**
Erfinder: **Merk, Wolfgang, Dr. Dipl.-Chem., Grünaustrasse 19, D-6450 Hanau 9 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft neue Bismelamine und die Verwendung von Bismelaminen für die Stabilisierung von Formaldehyd-Lösungen.

Es sind Alkylenbismelamine der Formel

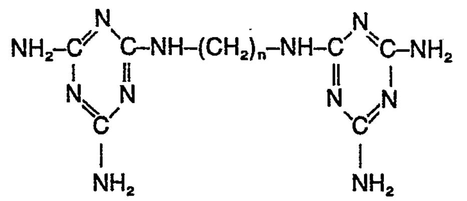

in der n eine Zahl von 1 bis 10 ist, bekannt (US-PS 2 544 071; J. Amer. Chem. Soc. 73 (1951), 2985). Die DE-A-2 625 399 betrifft härtbare Polyurethanmassen, die als Härtungsmittel u.a. das entsprechende Alkylenbismelamin enthält, in der n = 12 ist.

Es sind nun Alkylenbismelamine der Formel

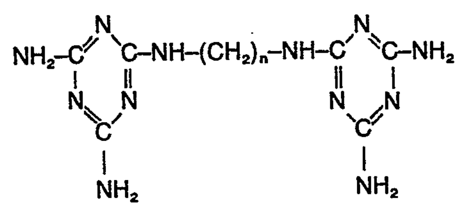

in der n eine Zahl von 11 bis 16 mit Ausnahme von n = 12 ist, gefunden worden.

Die erfindungsgemässen Alkylenbismelamine können in gleicher Weise wie die bekannten Alkylenbismelamine hergestellt werden, beispielsweise durch Umsetzung der entsprechenden aliphatischen Diamine mit Diaminochlortriazin, entsprechend dem Verfahren nach der US-PS 2 544 071 oder nach J. Amer. Chem. Soc. 73 (1951), 2985.

Zum Beispiel wird für die Herstellung des Decamethylenbismelamins von 1,10-Diaminodecan, für die Herstellung des Dodecamethylenbismelamins von 1,12-Diaminododecan und für die Herstellung des Tetradecamethylenbismelamins von 1,14-Diaminotetradecan ausgegangen.

Wässrige Formaldehyd-Lösungen, insbesondere Lösungen mit Gehalten von mehr als 30 Gewichtsprozent Formaldehyd, werden instabil, wenn bei der Lagerung bestimmte Temperaturen unterschritten werden. Es tritt Trübung durch Formaldehydoligomere und schliesslich Ausfällung von Paraformaldehyd auf. Die Lösungen sind umso instabiler, je höher dei Konzentration an Formaldehyd und je niedriger die Lagertemperatur ist. Nach den Angaben in der Monographie «Formaldehyde» von J.F. Walker, 3. Auflage, Seite 95, bleibt eine 30%ige Formaldehyd-Lösung bis etwa drei Monate lang stabil, wenn sie auf wenigstens 7 °C gehalten wird. Für eine 37%ige Lösung beträgt die erforderliche Mindesttemperatur 35 °C, für eine 45%ige Lösung 55 °C und für eine 50%ige Lösung 65 °C. Nachteilig ist bei der Anwendung hoher Lagertemperatur jedoch, dass sich in den Formaldehyd-Lösungen in erheblichem Umfang Ameisensäure bildet. Diese bewirkt Korrosionen und insbesondere stört sie bei der Verwendung der Formaldehyd-Lösungen zu Kondensationsreaktionen.

Die vorgenannten Werte beziehen sich auf Formaldehyd-Lösungen, die weniger als 1 Gewichtsprozent Methanol als Stabilisator enthalten. Bei Anwendung höherer Methanol-Konzentrationen, können zwar gleiche Lagerhaltbarkeiten bei niedrigeren Temperaturen erzielt werden, jedoch bedarf es unverhältnismässig hoher Methanol-Konzentrationen. Beispielsweise ist in einer 37%igen Formaldehyd-Lösung für eine Lagertemperatur von 21 °C ein Methanolgehalt von 7%, für 7 °C ein solcher von 10% und für 6 °C ein solcher von 12% nötig. Der Methanolzusatz verteuert die Formaldehyd-Lösungen erheblich, zumal das Methanol bei der Verwendung der Lösungen im allgemeinen verlorengeht. Abgesehen davon wird durch Methanol die Umsetzungsgeschwindigkeit bei zahlreichen Kondensationsreaktionen, beispielsweise bei der Kondensation mit Melamin, verringert.

Ausser Methanol sind als Stabilisatoren Äthanol, Propanol-(1), Propanol-(2), Glykol, Glycerin, Harnstoff, Methyl- und Dimethylharnstoff, Thioharnstoff, Diäthylthioharnstoff, Formamid, Melamin, Methylolmelamin und Acetoxim bekannt (J.F. Walker, «Formaldehyde», 3. Auflage, Seite 95; US-PS 2 002 243, 2 000 152 und 2 237 092). Diese Substanzen müssen jedoch, damit sie wirksam sind, in Konzentrationen von mindestens 2% angewendet werden.

Auch werden als Stabilisatoren 2,4-Diaminotriazine-(1,3,5) oder deren Methylolderivate angewendet, die in 6-Stellung einen Phenylrest oder einen aliphatischen Rest mit 7 bis 9 Kohlenstoffatomen tragen, insbesondere das Caprinoguanamin (DE-AS 1 205 071 und 1 205 073). Die Wirkung dieser Stabilisatoren wird gesteigert, indem zusätzlich hydrophile Polyglykoläther von Fettalkoholen oder von Polyalkohol-Fettsäurepartialestern eingesetzt werden (DE-AS 2 138 309). Jedoch ist auch in diesen Fällen die Wirkung der Stabilisatoren nicht befriedigend. Überdies neigen die so stabilisierten Formaldehyd-Lösungen stark zum Schäumen.

Es ist weiterhin bekannt, Phenylenbisguanamine als Stabilisatoren einzusetzen (DE-AS 2 358 856). Diese Substanzen zeigen zwar eine brauchbare Wirkung, sind jedoch verhältnismässig schwer zugänglich und weisen insbesondere den Nachteil auf, dass sie schwer löslich sind. Es ist daher schwierig und erfordert viel Zeit, die erforderliche Menge Stabilisator in löslicher Form einzubringen.

Schliesslich ist bekannt, als Stabilisatoren Alkylenbisguanamine anzuwenden (DE-OS 2 919 496). Zwar haben diese eine brauchbare Stabilisierwirkung und sind in den Formaldehyd-Lösungen gut löslich und daher leicht zu handhaben, sie sind jedoch schwer zugänglich.

Es ist nun ein Verfahren zur Stabilisierung von Formaldehyd-Lösungen gefunden worden, das dadurch gekennzeichnet ist, dass als Stabilisierungsmittel Alkylenbismelamine der Formel

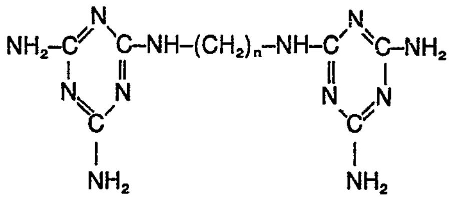

in der n eine Zahl von 10 bis 16 ist, eingesetzt werden. Diese Verbindungen zeigen bei gleicher Konzentration eine bessere Wirkung als alle bisher für diesen Zweck verwendeten Substanzen. Die Alkylenbismelamine sind verhältnismässig gut löslich und daher leicht zu handhaben.

Erfindungsgemäss werden als Stabilisatoren die Alkylenbismelamine eingesetzt, bei denen gemäss Formel n eine Zahl von 10 bis 16, vorzugsweise eine Zahl von 11 bis 14, insbesondere die Zahl 12, ist.

In welchen Mengen die Stabilisatoren den Formaldehyd-Lösungen zugesetzt werden, richtet sich gegebenenfalls in gewissem Umfang nach den Formaldehyd-Gehalten und den Lagertemperaturen der Lösungen. In den meisten Fällen kommen Stabilisator-Gehalte zwischen 0,001 und 0,1 Gewichtsprozent in Frage. Vorzugsweise werden Stabilisator-Gehalte zwischen 0,005 und 0,05, insbesondere zwischen 0,01 und 0,03 Gewichtsprozent gewählt.

Beispiele

A. Herstellung der Alkylenbismelamine

1. Einer Aufschlämmung von 291 g (2,0 Mol) 2,4-Dichlor-(6)-aminotriazin-(1,3,5) in 500 ml Wasser wurden 200 g (1,0 Mol) 1,12-Diaminododecan zugesetzt. Durch Zugabe einer 50%igen wässrigen Natriumhydroxyd-Lösung wurde der pH-Wert der Mischung auf 8,5 eingestellt. Die Mischung wurde unter Rühren zum Sieden erhitzt und 2,5 Stunden lang unter Rückfluss auf Siedetemperatur gehalten, dann abgekühlt und filtriert. Der Filterrückstand wurde so lange mit Wasser gewaschen, bis er frei von Chlorionen war. Es verblieben 388 g reines Dodecamethylenbismelamin entsprechend einer Ausbeute von 95%. Der Schmelzpunkt (Zersetzungspunkt) der Substanz war 182 bis 185 °C. Die Elementaranalyse ergab (in Klammern die für $C_{18}H_{34}N_{12}$ berechneten Werte): 52,1 (51,7) % C; 8,0 (8,2) % H; 39,9 (40,2) % N. Das Dodecamethylenbismelamin wurde ausserdem IR- und NMR-spektroskopisch sowie massenspektrographisch identifiziert.

2. Es wurde wie nach Beispiel 1 verfahren, jedoch wurden 228 g (1,0 Mol) 1,14-Diaminotetradecan eingesetzt. Es wurden 397 g Tetradecamethylenbismelamin gewonnen, entsprechend 91% Ausbeute. Der Schmelzpunkt (Zersetzungspunkt) der Substanz war 150 °C. Die Elementaranalyse ergab (in Klammern die für $C_{20}H_{38}N_{12}$ berechneten Werte): 54,3 (53,8) % C; 8,2 (8,8) % H; 37,5 (37,6) % N. Das Tetradecamethylenbismelamin wurde ausserdem IR- und NMR-spektroskopisch sowie massenspektrographisch identifiziert.

B. Stabilisierung der Formaldehyd-Lösungen

Es wurden Formaldehyd-Lösungen mit verschiedenden Gehalten an Formaldehyd verwendet. Diesen Lösungen wurden verschiedene Mengen an Bismelaminen und anderen Substanzen als Stabilisatoren zugesetzt und es wurde geprüft, wie lange diese Lösungen bei bestimmter Lagertemperatur stabil waren.

Zur Auflösung der Stabilisatoren in den Formaldehydlösungen wurden diese jeweils 20 bis 30 Minuten lang, im Falle des Isophthalobisguanamins 120 Minuten lang, unter Rühren auf 80 °C gehalten.

Die Ergebnisse sind in den folgenden Tabellen zusammengestellt. Die Stabilisator-Gehalte sind in Gewichtsprozenten, bezogen auf die Gesamt-Formaldehyd-Lösung angegeben. Als Lagerhaltbarkeit galt die Zeit, in der die Lösung stabil war. Die Lösungen wurden so lange als stabil angesehen, bis die erste gerade wahrnehmbare Abscheidung auftrat.

Tabelle 1

Lösungen mit 37 Gewichtsprozent Formaldehyd und 0,40 Gewichtsprozent Methanol.

| Stabilisator Art | Gehalt % | Lagertemperatur °C | Lagerhaltbarkeit Tage |
|---|---|---|---|
| Decamethylenbismelamin | 0,01 | 0 | 12 |
| Dodecamethylenbismelamin | 0,01 | 0 | 28 |
| Tetradecamethylenbismelamin | 0,01 | 0 | 30 |
| Caprinoguanamin | 0,01 | 0 | 4 |
| Dodecanobisguanamin | 0,01 | 0 | 10 |
| Isophthalobisguanamin | 0,01 | 0 | 10 |

Tabelle 2

Lösungen mit 40 Gewichtsprozent Formaldehyd und 0,50 Gewichtsprozent Methanol.

| Stabilisator Art | Gehalt % | Lagertemperatur °C | Lagerhaltbarkeit Tage |
|---|---|---|---|
| Decamethylenbismelamin | 0,03 | 0 | 6 |
| Dodecamethylenbismelamin | 0,03 | 0 | 15 |
| Tetradecamethylenbismelamin | 0,03 | 0 | 18 |
| Caprinoguanamin | 0,03 | 0 | 4 |
| Dodecanobisguanamin | 0,03 | 0 | 8 |
| Isophthalobisguanamin | 0,03 | 0 | 7 |

Tabelle 3

Lösungen mit 50 Gewichtsprozent Formaldehyd und 0,55 Gewichtsprozent Methanol.

| Stabilisator Art | Gehalt % | Lager-temperatur °C | Lagerhaltbarkeit Tage |
|---|---|---|---|
| Decamethylen-bismelamin | 0,015 | 39 | 8 |
| Dodecamethylen-bismelamin | 0,010 | 39 | 12 |
| | 0,015 | 39 | 30 |
| | 0,020 | 35 | 60 |
| Tetradecamethy-lenbismelamin | 0,010 | 39 | 16 |
| | 0,015 | 39 | 30 |

**Patentansprüche**

1. Alkylenbismelamine der Formel

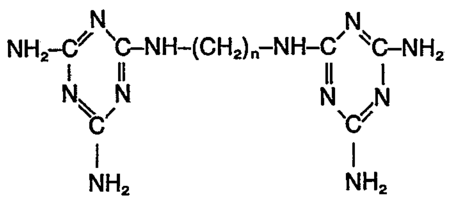

n = 11 bis 16, mit Ausnahme von n = 12.

2. Wässrige Formaldehyd-Lösungen, stabilisiert mit Melaminen, dadurch gekennzeichnet, dass sie Alkylenbismelamine der Formel

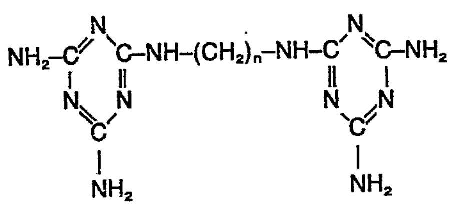

in der n eine Zahl von 10 bis 16 ist, enthalten.

3. Lösungen nach Anspruch 2, dadurch gekennzeichnet, dass sie 0,001 bis 0,1 Gewichtsprozent der Alkylenbismelamine enthalten.

4. Verwendung von Alkylenbismelaminen der Formel

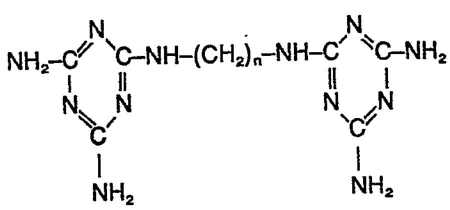

in der n eine Zahl von 10 bis 16 ist, zum Stabilisieren wässriger Formaldehyd-Lösungen.

**Claims**

1. Alkylene bismelamines corresponding to the formula

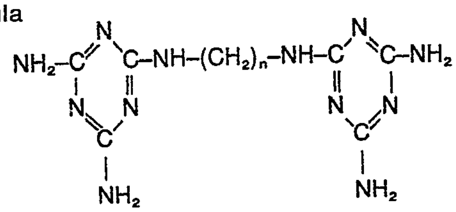

n = 11 to 16, with the exception of n = 12.

2. Aqueous formaldehyde solutions stabilized with melamines, characterised in that they contain alkylene bismelamines corresponding to the formula

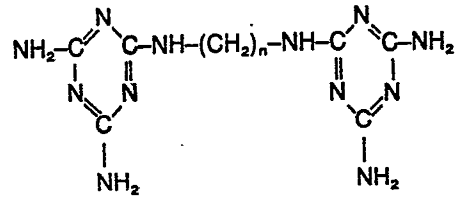

in which n is a number from 10 to 16.

3. Solutions according to claim 2, characterised in that they contain from 0.001 to 0.1% by weight of the alkylene bismelamines.

4. Use of alkylene bismelamines corresponding to the formula

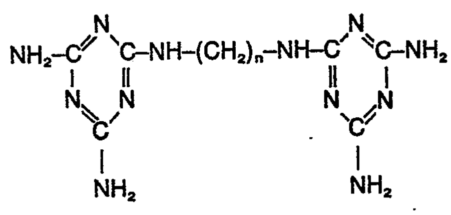

in which n is a number from 10 to 16, for the stabilization of aqueous formaldehyde solutions.

**Revendications**

1. Alkylènebismélamine répondant à la formule:

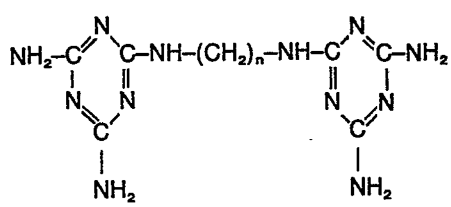

où n = 11 à 16, exception faite de n = 12.

2. Solutions aqueuses de formol, stabilisées avec des mélamines, caractérisées en ce qu'elles contiennent des alkylènebismélamines répondant à la formule:

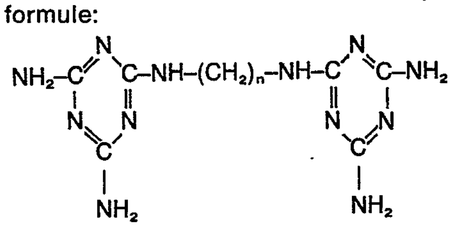

où n est un nombre de 10 à 16.

3. Solutions suivant la revendication 2, caractérisées en ce qu'elles contiennent de 0,001 à 0,1% en poids de l'alkylènebismélamine.

4. Utilisation des alkylènebismélamines répondant à la formule:

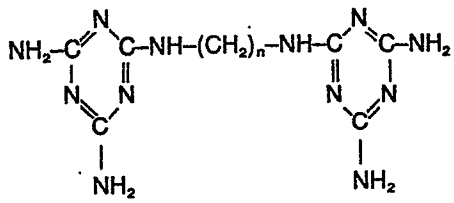

où n est un nombre de 10 à 16, pour la stabilisation de solutions de formol.